(19) Europäisches Patentamt
European Patent Office
Office européen des brevets

(11) Veröffentlichungsnummer: **0 044 801**
**B1**

(12) # EUROPÄISCHE PATENTSCHRIFT

(45) Veröffentlichungstag der Patentschrift: **15.02.84**

(51) Int. Cl.[3]: **C 07 C 59/01**, C 07 C 51/41, A 61 K 31/19

(21) Anmeldenummer: **81730055.1**

(22) Anmeldetag: **19.06.81**

(54) **Nichthygroskopische Salze der 4-Hydroxybuttersäure, Verfahren zu ihrer Herstellung und ihre Verwendung zur Herstellung pharmazeutischer Mittel.**

(30) Priorität: **17.07.80 DE 3027390**
**20.11.80 DE 3049869**

(43) Veröffentlichungstag der Anmeldung: **27.01.82 Patentblatt 82/4**

(45) Bekanntmachung des Hinweises auf die Patenterteilung: **15.02.84 Patentblatt 84/7**

(84) Benannte Vertragsstaaten: **AT BE CH FR GB IT LI LU NL SE**

(56) Entgegenhaltungen:
**GB - A - 922 029**

**"Beilsteins Handbuch der Organischen Chemie", 4. Auflage 1921, VERLAG VON JULIUS SPRINGER, Berlin Band 3, Seite 311**
**Chemical Abstracts Band 80, Nr. 19, 1974 Columbus, Ohio, USA A.T. ADVANI et al. "Formation constants of the proton and calcium complexes of prostaglandins PGE1 and PGF1beta" Seite 136, Spalte 2, Abstract Nr. 105125z**

(73) Patentinhaber: **Klosa, Josef, Dr., Jänickestrasse 13, D-1000 Berlin 37 (DE)**

(72) Erfinder: **Klosa, Josef, Dr., Jänickestrasse 13, D-1000 Berlin 37 (DE)**

(74) Vertreter: **Müller-Börner, Richard, Dipl.-Ing. et al, Patentanwälte Müller-Börner, Wey & Körner Podbielskiallee 68, D-1000 Berlin-Dahlem 33 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Nichthygroskopische Salze der 4-Hydroxybuttersäure, Verfahren zu ihrer Herstellung und ihre Verwendung zur Herstellung pharmazeutischer Mittel

4-Hydroxybuttersäure ist bekanntlich flüssig und nur schwer in reiner Form erhältlich. Sie ist auch in freier Form nicht im Handel. Die Natriumsalze der 4-Hydroxybuttersäure sind hygroskopisch. Ebenso sind die aus der GB-PS 922 029 bekannten Calciumsalze der 4-Hydroxybuttersäure auch nur in Form von Lösungen als anwendbar beschrieben. Ein Schmelzpunkt ist für das Calciumsalz nicht angegeben, da er offenbar infolge der starken Hygroskopizität nicht exakt bestimmbar ist. Gemäss der GB-PS 922 029 wird das hygroskopische Calciumsalz durch Umsetzen von γ-Butyrolacton mit Kalkmilch erhalten. Dies ist die allgemeine Arbeitsweise, aber die Reaktion verläuft auch äusserst stürmisch und stark exotherm, so dass es zu Nebenreaktionen kommen kann, die nicht kristallisierbare bis schwer kristallisierbare Endprodukte zeitigen.

Völlig unbekannt und bisher nicht beschrieben sind die Magnesiumsalze der 4-Hydroxybuttersäure. Damit ist eine breitere und allgemeinere therapeutische Anwendbarkeit der 4-Hydroxybuttersäure und ihrer Salze, wie in Form von Tabletten, Dragees oder Kapseln, bisher ausgeschlossen. Infolge der schwierigen Herstellung und Handhabung der 4-Hydroxybuttersäure und ihrer Salze sind diese auch sehr teuer.

Überraschenderweise wurde nun gefunden, dass sowohl die wasserfreien Calciumsalze der 4-Hydroxybuttersäure als auch die Magnesiumsalze dieser Säure nicht hygroskopisch und beliebig lange lagerfähig sind. Gegenstand der Erfindung sind somit nichthygroskopische Salze der 4-Hydroxybuttersäure der allgemeinen Formel I

$$HO-CH_2-CH_2-CH_2-COO-E-OOC-CH_2-CH_2-CH_2-OH \cdot x\, H_2O,$$

in der E = Calcium oder Magnesium und x = 0, 4, 5 oder 6 ist, mit der Massgabe, dass im Falle von E = Calcium x = 0 ist; Verfahren zu ihrer Herstellung, dadurch gekennzeichnet, dass man γ-Butyrolacton mit einem Hydroxid, Oxid oder Carbonat des Calciums oder Magnesiums nach dem Schema

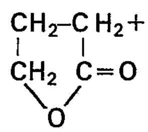 $+ E(OH)_2 \rightarrow (HO-CH_2-CH_2-CH_2-COO)_2E \cdot x\, H_2O,$

in dem E und x die im Anspruch 1 angegebene Bedeutung haben, hydrolytisch aufspaltet, wobei bei Einsatz von Calciumhydroxid, -oxid oder- carbonat bei Temperaturen unterhalb 80 °C, vorzugsweise bei 40 bis 60 °C, gearbeitet und die gegebenenfalls erhaltene hygroskopische Kristallmasse aus wässrigen Alkoholen umkristallisiert wird, und wobei bei Einsatz von Magnesiumhydroxid, -oxid oder -carbonat bei der Siedetemperatur des Wassers gearbeitet wird; und die Verwendung dieser Salze gegebenenfalls zusammen mit anderen Wirksubstanzen und/oder üblichen Füll- und Trägerstoffen, zur Herstellung eines pharmazeutischen Mittels.

Das wasserfreie Calciumsalz der Formel I hat einen konstanten Schmelzpunkt von 166 bis 168 °C und zieht selbst nach stundenlangem Lagern an der Luft kein Wasser an, d.h. es bleibt völlig trokken. Es wurde festgestellt, dass es bereits dann kristallin erhalten werden kann, wenn man γ-Butyrolacton und Calciumhydroxid, -oxid oder -carbonat in der Weise umsetzt, dass die Temperatur unterhalb 80 °C, vorzugsweise zwischen 40 und 60 °C, bleibt.

Angesichts der Tatsache, dass bekannt war, dass wasserhaltige Calciumsalze der 4-Hydroxybuttersäure hygroskopisch sind, war es um so überraschender, festzustellen, dass die Magnesiumsalze dieser Säure, die bisher noch nicht beschrieben waren, sowohl in wasserfreiem als auch in wasserhaltigem Zustand nicht hygroskopisch sind. Die Magnesiumsalze der Formel I können bis zu maximal 6 Mole Kristallwasser binden. Nach kurzer Trocknung an der Luft vermindert sich der Kristallwassergehalt auf 4 bis 5 Mole. Alle diese Magnesiumsalze sind nicht hygroskopisch, gut lagerfähig und zur Bereitung verschiedener pharmazeutischer Mittel ebenso gut geeignet wie das oben erläuterte wasserfreie Calciumsalz.

Es ist bekannt, dass 4-Hydroxybuttersäure narkotisch wirkt. In Form des Natriumsalzes wird sie als intravenös injizierbares Narkosemittel mit ausgezeichnetem Erfolg in der Medizin verwendet. Sie erzeugt einen von der Dosis abhängigen Schlafzustand, aus dem der Patient erweckbar ist. Vor allem entfaltet die 4-Hydroxybuttersäure keinerlei Giftwirkung oder Nebenwirkungen, welche den bekannten narkotischen oder sedativ wirkenden Mitteln anhaften. Sie wird im Organismus völlig abgebaut und ist auch mit den körpereigenen Stoffwechselprodukten, wie der Milchsäure, nahe verwandt. Vor allem belastet die 4-Hydroxybuttersäure nicht die Leber, wie es Sedativa und Hypnotika tun.

Jedoch waren die freie 4-Hydroxybuttersäure sowie ihr Natriumsalz und auch das aus der GB-PS 922 029 bekannte Calciumsalz wegen ihrer starken Hygroskopizität in breiterem Rahmen nicht pharmazeutisch anwendbar. Es besteht jedoch eine grosse Notwendigkeit für diese Mittel

insbesondere als Schlaf- und Beruhigungsmittel, weil praktisch alle bekannten Schlaf- und Beruhigungsmittel, wie die Barbiturate, Amide, aber auch Diazepame, bei Daueranwendung mit grossen Nebenwirkungen, aber auch direkt mit unliebsamen Nachwirkungen verbunden sind. Vor allem greifen alle gegenwärtigen Schlaf- und Beruhigungsmittel – das gilt auch für Pflanzenwirkstoffe, wie diejenigen des Baldrians – ungünstig in den Nervenstoffwechsel ein. Sie werden vor allem in nicht beherrschbare und unübersichtlich wirkende Metabolite umgewandelt und schädigen die Leber oft irreversibel.

Nun ist von der ungiftigen 4-Hydroxybuttersäure zwar bekannt, dass sie als Schlafmittel wirkt (vgl. G. Kuschinsky, Taschenbuch der modernen Arzneibehandlung, 8. Auflage, 1980, Seite 328), jedoch ist eine handliche orale Applikationsweise in Form von Tabletten, Dragees oder Kapseln wegen der starken Hygroskopizität der bisher bekannten diesbezüglichen Verbindungen nicht möglich gewesen. Aber selbst in Form von Lösungen waren die bekannten Verbindungen nicht exakt dosierbar, da hygroskopische Substanzen immer schnell Wasser anziehen, sogar beim Wägen, so dass bei Grossherstellung von pharmazeutischen Mitteln konstante Dosen nicht eingehalten werden können.

Diese Schwierigkeiten werden durch die erfindungsgemässen nichthygroskopischen Salze der 4-Hydroxybuttersäure ausgeräumt. Es sind aus ihnen mühelos feste wie flüssige Darreichungsformen herstellbar, auch entfallen bei ihnen die Nachteile der Lösungen von freier Säure und Natriumsalz, die ätzend und brennend auf die Mundschleimhaut wirken.

Es hat sich herausgestellt, dass die nichthygroskopischen Calcium- und Magnesiumsalze der 4-Hydroxybuttersäure gemäss der Erfindung in Dosen von 100 bis 2000 mg, vorzugsweise von 300 bis 500 mg, oral als Tabletten, Dragees oder Kapseln verabreicht, eine ausgezeichnete Beruhigungs- und Schlafwirkung entfalten. In pharmakologischen Untersuchungen hat sich jedoch in erster Linie eine ausgezeichnete analgetische Wirkung der erfindungsgemässen Verbindungen gezeigt. So steigern sie in Kombination mit schmerzstillenden Mitteln, wie Salicylaten, z.B. Salicylamid, Acetylsalicylsäure oder p-Acetylaminophenol, deren analgetische Wirkung. Das gilt auch für die Kombination mit Phenylbutazon. Die erfindungsgemässen Verbindungen eröffnen neue Möglichkeiten der Schmerzbekämpfung, so als mögliche Schmerzstillalternative beim Analgetika-Asthma-Syndrom. Schmerzhafter Stress, wie er bei chronischen Schmerzen auftritt, wird vermindert und beseitigt. Günstig ist deshalb die Kombination der erfindungsgemässen Salze mit analgetischen und antirheumatisch wirksamen Arzneimitteln. Eine solche Kombination ist mit den erfindungsgemässen Salzen problemlos vornehmbar, im Gegensatz zu den bekannten hygroskopischen Substanzen, die in chemische Wechselwirkungen zu den Kombinationspartnern treten.

Die erfindungsgemässen Calcium- und Magnesiumsalze der 4-Hydroxybuttersäure sind fest, nicht hygroskopisch, haltbar und lassen sich umkristallisieren. Sie können mit Tablettenhilfsstoffen zu Tabletten gepresst, dragiert und als Pulver in Kapseln gefüllt werden. Sie lösen sich auch klar in Wasser und können in Form von Injektionslösungen, aber auch als Tropfen, in Getränken oder Zäpfchen unter Beibehaltung stabiler Verhältnisse verabreicht werden.

Die Erfindung wird nachfolgend anhand von Beispielen im einzelnen erläutert, die jedoch keinerlei Einschränkung des Erfindungsbereiches bedeuten.

Beispiel 1

In 86 g γ-Butyrolacton wird unter Rühren portionsweise eine Aufschlämmung von 37 g Calciumhydroxid in 80 ml Wasser zugefügt. Es tritt Erwärmung ein. Die Zugabe der wässrigen Calciumhydroxidaufschlämmung wird so geregelt, dass es nicht zum Sieden kommt. Nach Beendigung der Zugabe wird das farblose Reaktionsgut noch einige Stunden sich selbst überlassen. Gewöhnlich wird eine farblose Lösung erhalten, anderenfalls wird vom Niederschlag abdekantiert und die farblose, etwas sirupöse Lösung auf dem Wasserbad, eventuell bei vermindertem Druck, vom Wasser befreit. Der Rückstand kristallisiert zu einer farblosen Kristallmasse, die bei 60 bis 80 °C noch nachgetrocknet wird. Ausbeute: ca. 105 g. Fp. 164–166 °C. Die Analyse ergibt 16,21% Ca (berechnet 16,26% Ca). Das Produkt ist Di-(4-hydroxybuttersäure)-Calcium. Es wird durch Lösen in wenig Methanol und Zusatz von Aceton bis zur Trübung umkristallisiert.

Beispiel 2

74 g analysenreines Calciumhydroxid werden in 200 ml Leitungswasser suspendiert. In diese Suspension werden unter Rühren bei Raumtemperatur 160 ml γ-Butyrolacton portionsweise (in Portionen zu 5 bis 10 ml) einfliessen gelassen. Nach Zusatz von 20 ml wärmt sich das Reaktionsgut auf 50 bis 60 °C. Das Zufliessen des γ-Butyrolactons wird so geregelt, dass die Temperatur zwischen 50 und 60 °C bleibt, was etwa eine Stunde dauert. Das Calciumhydroxid hat sich in dieser Zeit praktisch vollständig gelöst. Das Reaktionsgut ist durch geringen rostgelben Niederschlag verunreinigt. Es wird mit 300 ml Methanol verdünnt, vier Stunden sich selbst überlassen und dann durch ein Faltenfilter filtriert. Das klare Filtrat wird vorsichtig mit 200 ml Aceton versetzt, und zwar in der Weise, dass man nach jeder Portion Aceton, die eine Fällung hervorruft, die Fällung sich wieder auflösen lässt. Man erhält eine wasserklare Lösung, die zum Kristallisieren gestellt wird. Nach zwei Stunden Stehen beginnen sich farblose Kristalle abzuscheiden. Die Kristallisation wird in diesem Zustand durch laufenden Zusatz von Aceton (insgesamt 100 ml) beschleunigt. Dauer der Kristallisation 24 Stunden. Die Kristalle werden abgesaugt und zuerst mit 50 ml Methanol und dann noch mit 60 ml Aceton gewaschen. Es wird bei 60 bis 80 °C im Trockenschrank getrocknet. Ausbeute: 230 g. Fp. 166–168 °C (sofort). Das Produkt ist das wasserfreie nichthygroskopische Calciumsalz der 4-Hydroxybuttersäure. Es ist in Wasser beliebig löslich, die wässrige Lösung hat einen pH-Wert von 7 bis 7,5. Das Salz ist beliebig lange lagerfähig und verändert sich nicht an der Luft. Es zieht selbst bei Lagerung an der Luft kein Wasser an.

Beispiel 3

Auf die in Beispiel 2 beschriebene Weise werden 74 g Calciumhydroxid mit 160 ml γ-Butyrolacton in 200 ml Wasser umgesetzt. Das sirupöse Reaktionsgut wird auf dem Wasserbad eingedampft und einige Tage stehengelassen, so dass es zu einer hygroskopischen Kristallmasse erstarrt. Diese wird zerkleinert und mit 300 ml Methanol unter Rühren auf dem Wasserbad erwärmt. Es wird erkalten gelassen, abgesaugt und mit Aceton getrocknet. Das rohe Calciumsalz der 4-Hydroxybuttersäure ist nicht hygroskopisch und schmilzt schwach bei 164–166 °C. Ausbeute: 204 g. Das Salz löst sich in Wasser glatt auf, jedoch mit flokkigen Verunreinigungen. Zur Reinigung wird das Rohprodukt in 500 ml Methanol und 80 ml Wasser gelöst und filtriert. Das wasserklare Filtrat wird nochmals mit 100 ml Methanol und dann unter Rühren portionsweise mit 400 ml Aceton versetzt. Es darf kein Niederschlag auftreten. Nach einigen Stunden beginnt die Kristallisation, die 24 bis 48 Stunden dauert. Die Kristalle werden abgesaugt, zuerst mit 60 ml Methanol, dann mit 100 ml Aceton gewaschen und bei 60 bis 80 bis 100 °C getrocknet. Ausbeute: 220 g in analysenreiner Beschaffenheit. Fp. 166–168 °C.

Anstelle von Methanol können zum Umkristallisieren mit gleichem Erfolg auch Äthanol und Isopropanol verwendet werden. Ohne Verwendung von wässrigen Alkoholen als Umkristallisiermittel bzw. Hilfsmittel der Umkristallisation und Reinigung werden keine stabilen, vor allem keine nichthygroskopischen Calciumsalze erhalten. Der Anteil an Wasser in den Alkoholen soll 3 bis 10% betragen.

Das obige reine Endprodukt ist leicht löslich in Wasser, nicht hygroskopisch und von angenehmem, aromatischem Geruch.

Beispiel 4

In 86 g γ-Butyrolacton werden unter Rühren und Erwärmen auf dem Wasserbad auf 50 bis 60 °C 42 g Magnesiumcarbonat in 100 ml Wasser einfliessen gelassen. Es entwickelt sich Kohlendioxid. Nach Beendigung der Zugabe wird auf dem Wasserbad bis zur vollständigen Auflösung noch 3 bis 5 Minuten lang erhitzt. Daraufhin wird die wasserklare Lösung in einer Schale auf dem Wasserbad eingedampft. Man erhält einen farblosen Sirup, der erstarrt, wenn er 14 Tage lang stehengelassen wird. Die Kristallmasse in einer Ausbeute von 120 g enthält noch Kristallwasser (Fp: 105–107 °C unter Aufbrausen), welches durch Trocknen bei 60 bis 80 °C entfernt werden kann. Fp. 172–174 °C. Bei der Analyse wurden 10,47% Mg gefunden (berechnet 10,55% Mg). Das Produkt ist das Magnesiumsalz der 4-Hydroxybuttersäure. Summenformel: $C_8H_{14}O_6Mg$ (Mg 230,39). Es ist gut löslich in Wasser, Methanol und Äthanol, unlöslich in Äther und Kohlenwasserstoffen, nicht hygroskopisch, haltbar und von angenehmem aromatischem Geruch.

Beispiel 5

60 g Magnesiumhydroxid (p.a.) werden in 200 ml Leitungswasser unter Rühren suspendiert. In diese Suspension werden in einem Strahl unter Rühren 160 ml γ-Butyrolacton eingerührt. Nun wird unter Rühren in einem 2-1-Kolben sechs Stunden auf dem Wasserbad erwärmt. Das Magnesiumhydroxid ist praktisch vollständig in Lösung gegangen. Den Kolben lässt man über Nacht stehen, wobei sich Verunreinigungen abscheiden, von denen mühelos abdekantiert werden kann. Das wasserklare Dekantat wird zuerst zehn Minuten lang mit 100 ml Aceton durchgerührt. Die farblose sirupöse Flüssigkeit, die nun zäher geworden ist, wird noch einmal mit 100 ml Aceton, wie vorher, durchgeführt, das Aceton durch Dekantieren erneut entfernt und der ziemlich zähe, farblose Sirup zwei bis vier Stunden bei Raumtemperatur sich selbst überlassen. Er erstarrt zu einer farblosen Kristallmasse, die im Mörser zerkleinert und einige Stunden an der Luft getrocknet wird. Fp. 76–78 °C. Ausbeute: 314 g in analysenreiner Form.

Summenformel: $C_8H_{14}O_6Mg \cdot 5_2O$. Dieses Magnesiumsalz enthält ca. 5 Mole Kristallwasser. Es ist nicht hygroskopisch, stabil und beliebig lange lagerfähig. Durch mehrstündiges Trocknen bei 40 bis 50 °C verliert es einen Teil Kristallwasser (1 Mol) und schmilzt dann bei 118 bis 120 °C. Das wasserfreie Salz schmilzt bei 172–174 °C. Die Analyse ergibt 10,50% Mg (berechnet 10,55% Mg).

Alle Modifikationen sind nicht hygroskopisch und lagerfähig. 1 g des Magnesiumsalzes löst sich in 2 ml Wasser bei Raumtemperatur auf; der pH-Wert der wässrigen Lösung beträgt 7.

Beispiel 6

Herstellung von Tabletten:

| | |
|---|---|
| Di(4-hydroxybuttersäure)-Calcium | 500 mg |
| Tablettenhilfsmittel (nach konventioneller Weise) | ad 1000 mg |

Beispiel 7

Herstellung von Kapseln:

| | |
|---|---|
| Di(4-hydroxybuttersäure)-Magnesium | 300 mg |
| Laktose | 120 mg |
| Mikrokristalline Zellulose | 30 mg |
| Ärosil-200 (eingetragenes Warenzeichen der Firma DEGUSSA) | 2 mg |
| Magnesiumstearat | 3 mg |
| Gewicht der Kapsel: | 455 mg |

Beispiel 8

Herstellung von Suppositorien:

| | |
|---|---|
| Di(4-hydroxybuttersäure)-Calcium | 500 mg |
| Suppositorienmasse | 1500 mg |
| Gewicht des Supp.: | 2000 mg |

Beispiel 9
Herstellung von Honigflüssigkeit:

In 80 g Bienenhonig wurden 10 g Magnesiumsalz nach Beispiel 2 eingeführt. Es wurde ein gelber Honigsirup von wohlschmeckender Beschaffenheit erhalten.

Beispiel 10
Tabletten gegen Schmerzen:

| | |
|---|---|
| Di(4-hydroxybuttersäure)-Calcium | 100 mg |
| Salicylamid | 300 mg |
| Tablettenhilfsstoffe ad | 1000 mg |

Beispiel 11
Herstellung von Dragees:

| | |
|---|---|
| Di(4-hydroxybuttersäure)-Calcium | 200 mg |
| Phenylbutazon | 100 mg |
| Drageehilfsmittel | ad 600 mg |

Beispiel 12
Herstellung von Kapseln:

| | |
|---|---|
| Di(4-hydroxybuttersäure)-Calcium | 200 mg |
| Acetylsalicylsäure | 250 mg |
| Sorbit | 50 mg |
| Gesamt: | 500 mg |

**Patentansprüche**

1. Nichthygroskopische Salze der 4-Hydroxybuttersäure der allgemeinen Formel

$$HO-CH_2-CH_2-CH_2-COO-E-OOC-CH_2-CH_2-CH_2-OH \cdot x\,H_2O,$$

in der E = Calcium oder Magnesium und x = 0, 4, 5 oder 6 ist, mit der Massgabe, dass im Falle von E = Calcium x = 0 ist.

2. Verfahren zur Herstellung der nichthygroskopischen Salze nach Anspruch 1, dadurch gekennzeichnet, dass man γ-Butyrolacton mit einem Hydroxid, Oxid oder Carbonat des Calciums oder Magnesiums nach dem Schema

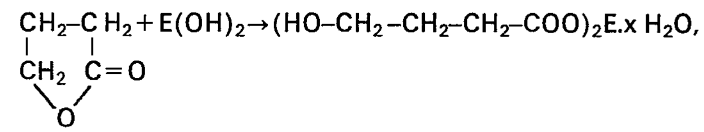

in dem E und x die im Anspruch 1 angegebene Bedeutung haben, hydrolytisch aufspaltet, wobei bei Einsatz von Calciumhydroxid, -oxid oder -carbonat bei Temperaturen unterhalb 80 °C, vorzugsweise bei 40 bis 60 °C, gearbeitet und die gegebenenfalls erhaltene hygroskopische Kristallmasse aus wässrigen Alkoholen umkristallisiert wird, und wobei bei Einsatz von Magnesiumhydroxid, -oxid oder -carbonat bei der Siedetemperatur des Wassers gearbeitet wird.

3. Verwendung von nichthygroskopischen Salzen nach Anspruch 1, gegebenenfalls zusammen mit anderen Wirksubstanzen und/oder üblichen Füll- und Trägerstoffen, zur Herstellung eines pharmazeutischen Mittels.

**Revendications**

1. Sels non hygroscopiques de l'acide 4-hydroxybutyrique, correspondant à la formule générale:

$$HO-CH_2-CH_2-CH_2-COO-E-OOC-CH_2-CH_2-CH_2 \cdot x\,H_2O$$

dans laquelle le symbole E représente le calcium ou le magnésium, le symbole x pouvant avoir l'une des valeurs 0, 4, 5 ou 6 et ayant la valeur x = 0 si E = calcium.

2. Procédé pour la préparation des sels hygroscopiques définis dans la revendication 1, caractérisé en ce qu'on opère à partir de la γ-butyrolactone, pour soumettre cette substance à un traitement de cracking sous l'effet d'un hydroxyde, d'un oxyde, ou d'un carbonate de calcium ou de magnésium, suivant le schéma:

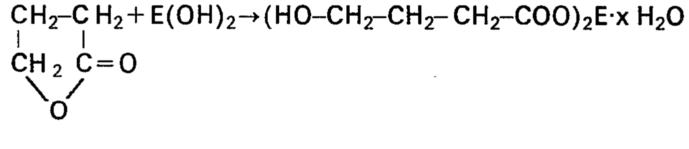

dans lequel les symboles E et x ont la signification indiquée dans la revendication 1; en ce que le traitement en présence d'hydroxyde, d'oxyde, ou de carbonate de calcium s'effectue à des températures inférieures à 80 °C, et de préférence entre 40 et 60 °C; en ce qu'on recristallise au moyen d'une solution aqueuse d'alcools la masse de cristaux hydroscopiques éventuellement obtenue; et en ce qu'on opère à la température d'ébullition de l'eau, lorsqu'on effectue le traitement au moyen d'hydroxyde, d'oxyde, ou de carbonate de magnésium.

3. Utilisation de sels non hygroscopiques tels que définis à la revendication 1, éventuellement avec d'autres substances actives, et/ou avec des substances couramment employées comme supports ou matières de remplissage, en vue de la préparation d'un produit pharmaceutique.

## Claims

1. Nonhygroscopic salts of the 4-hydroxybutyric acid of the general formula

$$HO{-}CH_2{-}CH_2{-}CH_2{-}COO{-}E{-}OOC{-}CH_2{-}CH_2{-}CH_2{-}OH \cdot x\ H_2O$$

where E = calcium or magnesium and x = 0, 4, 5 or 6 on the condition that in case of E = calcium x = 0.

2. A method for the production of the nonhygroscopic salts according to claim 1, characterized by the fact that γ-butyrolactone with a hydroxyde, oxyde or carbonate of the calcium or magnesium is hydrolytically split up according to the schema

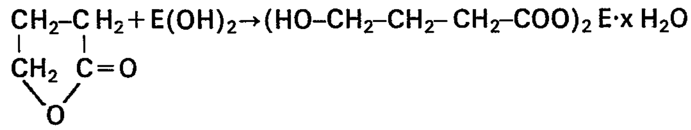

where E and x have the meaning as in claim 1, whereby when using calcium hydroxyde, calcium oxyde or calcium carbonate it is worked at temperatures below 80 °C, preferably at 40 or 60 °C, and the possibly obtained hygroscopic crystal mass is recrystallized out of aqueous alcohols and whereby in case of use of magnesium hydroxy, magnesium oxyde or magnesium carbonate it is worked at the boiling temperature of water.

3. Use of nonhygroscopic salts according to claim 1, if indicated together with other additives and/or usual filler or carrier material for production of pharmaceutical preparations.